# EUROPÄISCHE PATENTANMELDUNG

(11) **EP 3 708 940 A1**
(43) Veröffentlichungstag der Anmeldung: **16.09.2020**
(21) Anmeldenummer: 20162740.3
(22) Anmeldetag: 12.03.2020
(51) Int. Cl.: F28D 9/00, F28F 3/04, A61F 7/00, A61M 5/44, A61M 1/30

(54) **VORRICHTUNG UND VERFAHREN ZUR TEMPERIERUNG**

(30) Priorität: 15.03.2019 DE 102019106713
(71) Anmelder: Lauda Dr. R. Wobser GmbH & Co. KG, 97922 Lauda-Königshofen (DE)
(72) Erfinder: Eller, Mathias, 97237 Altertheim (DE)
(74) Vertreter: Zimmermann & Partner Patentanwälte mbB

(57) **Zusammenfassung**

Vorrichtung (100, 201) zur Temperierung, insbesondere zur extrakorporalen Temperierung von Patienten, mit einer Übertragung von Wärme zwischen einem Fluid und einem Übertragungsmedium, mit einem Temperierkörper (203) mit einer Primärseite, welche eine Führung für das Fluid bereitstellt, und mit einer Applikationsseite, welche eine Oberfläche (219) zum Kontakt des Temperierkörpers (203) mit dem Übertragungsmedium bereitstellt; einer an dem Temperierkörper (203) befestigten Abdeckung (221), welche an der Applikationsseite mit dem Temperierkörper (203) einen Hohlraum bildet, der von dem Übertragungsmedium durchströmt werden kann; wobei die Abdeckung (221) lösbar an dem Temperierkörper (203) befestigt ist; und wobei an dem Temperierkörper (203) und/oder an der Abdeckung (221) strömungsführende Strukturen (217) zur Führung des Übertragungsmediums angeordnet sind.

## Beschreibung

### Gebiet der Erfindung

Die Erfindung betrifft eine Vorrichtung und ein Verfahren zur Temperierung, insbesondere zur extrakorporalen Temperierung von Patienten und zur extrakorporalen Temperierung von Körperflüssigkeiten.

### Stand der Technik

Zur extrakorporalen Temperierung von Patienten sind Verfahren und Vorrichtungen bekannt, welche Wärmetauscher verwenden oder umfassen. Bei den Wärmetauschern wird in vielen Fällen eine Flüssigkeit auf Wasserbasis als Medium zum Transport von Wärme oder Kälte verwendet.

Allerdings haben bisher bekannte Lösungen aus dem Stand der Technik Restriktionen in Bezug auf die übertragbare Wärmeleistung, insbesondere in Bezug auf ein Gehäusevolumen des Wärmetauschers, auf die dauerhafte Erfüllung von Hygieneanforderungen oder sie sind nur aufwendig zu reinigen.

### Offenbarung der Erfindung

Der Erfindung liegt die Aufgabe zugrunde, eine verbesserte Vorrichtung zur Temperierung, insbesondere zur extrakorporalen Temperierung von Patienten oder von Körperflüssigkeiten anzugeben. Insbesondere sollte die Hygiene verbessert werden oder es sollte die übertragbare Wärmeleistung, insbesondere in Bezug auf einen Bauraum der Vorrichtung, verbessert werden oder der Aufbau sollte vereinfacht sein oder einfacher zu reinigen sein oder anderweitig die Einhaltung von Hygienestandards erleichtern.

Gemäß einem Aspekt der Erfindung ist eine Vorrichtung zur Temperierung, insbesondere zur extrakorporalen Temperierung von Patienten oder von Körperflüssigkeiten, mit einer Übertragung von Wärme zwischen einem Fluid und einem Übertragungsmedium angegeben, mit einem Temperierkörper mit einer Primärseite, welche eine Führung für das Fluid bereitstellt, und mit einer Applikationsseite, welche eine Oberfläche zum Kontakt des Temperierkörpers mit dem Übertragungsmedium bereitstellt; einer an dem Temperierkörper befestigten Abdeckung, welche an der Applikationsseite mit dem Temperierkörper einen Hohlraum bildet, der von dem Übertragungsmedium durchströmt werden kann; wobei die Abdeckung lösbar an dem Temperierkörper befestigt ist; und wobei an dem Temperierkörper oder an der Abdeckung strömungsführende Strukturen zur Führung des Übertragungsmediums angeordnet sind, welche typischerweise bei Befestigung der Abdeckung an dem Temperierkörper von der Oberfläche der Applikationsseite bis zu einer Deckplatte der Abdeckung reichen.

Gemäß einem weiteren Aspekt der Erfindung ist ein Verfahren zur Temperierung, insbesondere zur extrakorporalen Temperierung von Patienten oder von Körperflüssigkeiten unter Verwendung eines Temperierkörpers und zweier Abdeckungen, insbesondere entsprechend einer Vorrichtung nach einer der hierin beschriebenen Ausführungsformen angegeben, mit Befestigen der ersten Abdeckung an dem Temperierkörper, Verwenden der Vorrichtung zur Temperierung des Übertragungsmediums, Lösen der ersten Abdeckung von dem Temperierkörper, Befestigen der zweiten Abdeckung an dem Temperierkörper, Verwenden der Vorrichtung zur Temperierung des Übertragungsmediums. Typische Temperierkörper sind an der Primärseite des Temperierkörpers an einen Primärkreis angeschlossen, in welchem ein Fluid aufnehmbar ist. Typischerweise ist das Fluid eine Flüssigkeit oder ein Gas oder ein Gas-Flüssigkeitsgemisch, das zur Speicherung und Übertragung von Wärme geeignet ist, beispielsweise Wasser, Pflanzenöl, Ammoniak, Kohlenstoffdioxid, Kohlenwasserstoffe, Fluorkohlenwasserstoffe, anorganische Kältemittel oder Luft. Unter dem Begriff Wärme oder Übertragung von Wärme wird hierin auch Kälte oder die Übertragung von Kälte verstanden. Die Begriffe "Wärme" und "Übertragung von Wärme" werden hierin in einem physikalischen Sinn verstanden.

Typischerweise weist der Temperierkörper an der Applikationsseite des Temperierkörpers eine Oberfläche zum Kontakt des Temperierkörpers mit dem Übertragungsmedium und zur Übertragung von Wärme zwischen dem Temperierkörper und dem Übertragungsmedium auf. Das Übertragungsmedium ist typischerweise in einem Sekundärkreis aufgenommen. Typischerweise ist das Übertragungsmedium eine Flüssigkeit. Mit dem Sekundärkreis wird bei typischen Ausführungsformen eine Körperflüssigkeit extrakorporal temperiert. Bei typischen Ausführungsformen umfasst das Übertragungsmedium mindestens 90 Gew.-% oder mindestens 95 Gew.-% Wasser, insbesondere Wasser für Injektionszwecke (WFI). In einer typischen Ausführungsform ist das Übertragungsmedium ein physiologisches oder isotonisches Medium. Typischerweise ist das Übertragungsmedium steril oder weitgehend keimfrei, beispielsweise weniger als 1000 oder maximal 100 koloniebildende, aerobe Keime pro Liter. Physiologische oder isotonische Medien sind beispielsweise eine 0,9%ige Lösung mit Kochsalz (Natriumchlorid, NaCI) oder eine Ringer-Lösung.

Bei typischen Ausführungsformen der Vorrichtung bildet der Temperierkörper mit der an dem Temperierkörper befestigten Abdeckung an der Applikationsseite des Temperierkörpers einen Hohlraum. Typischerweise ist der Hohlraum, mit Ausnahme von Anschlüssen zur Zu- und Abführung des Übertragungsmediums, flüssigkeitsdicht abgeschlossen.

Typischerweise wird der Hohlraum umschlossen durch die zum Kontakt mit dem Übertragungsmedium vorgesehene Oberfläche der Applikationsseite des Temperierkörpers, durch eine der Oberfläche der Applikationsseite des Temperierkörpers gegenüberliegende, insbesondere durch eine zu der Oberfläche zumindest im Wesentlichen parallelen, Deckplatte der Abdeckung, und durch eine Randplatte, welche sich insbesondere zwischen den Rändern der Deckplatte der Abdeckung und der Oberfläche der Applikationsseite des Temperierkörpers erstreckt. Typischerweise ist die Randplatte als integraler Teil der Abdeckung ausgeführt. Typischerweise ist eine Abdeckung, die eine Deckplatte und eine Randplatte umfasst, als Haube auf die Oberfläche der Applikationsseite des Temperierkörpers gesetzt. In weiteren Ausführungsformen ist die Randplatte als Teil des Temperierkörpers ausgeführt. Bei weiteren Ausführungsformen ist die Deckplatte bezüglich der Oberfläche der Applikationsseite des Temperierkörpers gewölbt oder gekrümmt.

Bei typischen Ausführungsformen sind die strömungsführenden Strukturen integral mit dem Teil des Temperierkörpers hergestellt, der die Oberfläche der Applikationsseite bereitstellt, oder die strömungsführenden Strukturen sind nachträglich an der Oberfläche der Applikationsseite des Temperierkörpers angebracht. Bei weiteren typischen Ausführungsformen sind die strömungsführenden Strukturen als Teil der Abdeckung hergestellt oder nachträglich an der Deckplatte der Abdeckung angebracht. Bei typischen Ausführungsformen erstrecken sich zumindest ein Teil der strömungsführenden Strukturen zwischen der Oberfläche der Applikationsseite und der Deckplatte der Abdeckung über mindestens 50%, typischerweise über mindestens 70% oder über mindestens 90%, des Abstandes zwischen der Oberfläche der Applikationsseite und der Deckplatte der Abdeckung.

Bei typischen Ausführungsformen bilden der Temperierkörper und die Abdeckung durch die strömungsführenden Strukturen Kanäle, die vom Übertragungsmedium durchströmt werden können. Der Hohlraum, der von dem Temperierkörper und der Abdeckung gebildet wird, wird typischerweise durch strömungsführende Strukturen, beispielsweise als Rippen ausgeführt, in Kanäle gegliedert. Typischerweise verlaufen Kanäle parallel oder mäandernd. In typischen Ausführungsformen führen die strömungsführenden Strukturen das Übertragungsmedium parallel oder in Reihe durch mindestens 5, insbesondere durch mindestens 10 oder mindestens 20 Kanäle. Als Rippen ausgeführte strömungsführende Strukturen sind typischerweise als gerade oder als gewellte Rippen ausgeführt. Typischerweise reichen die Rippen von der Oberfläche des Temperierkörpers bis zu der Deckplatte der Abdeckung, wodurch ein Fluss von Übertragungsmedium zwischen zwei benachbarten Kanälen zumindest im Wesentlichen verhindert werden kann.

Bei typischen Ausführungsformen führen die strömungsführenden Strukturen eine Strömung des Übertragungsmediums in dem von dem Temperierkörper und der Abdeckung gebildeten Hohlraum zumindest teilweise parallel, anti-parallel oder mäandernd. Durch die Ausgestaltung der Innenseite, insbesondere die dem Übertragungsmedium zugewandte Seite der Abdeckung, können verschiedene Strömungsvarianten bereit gestellt werden. Alle Kanäle parallel, einige Kanäle parallel oder diese zumindest teilweise parallelen Kanäle mäandrierend oder ein einzelner mäandrierender Kanal.

Bei typischen Ausführungsformen weisen der Temperierkörper oder die Abdeckung Verwirbelungsstrukturen zur Verwirbelung des Übertragungsmediums auf. Verwirbelungsstrukturen sind typischerweise stromaufwärts oder stromabwärts von strömungsführenden Strukturen oder in Kanälen, die von strömungsführenden Strukturen gebildet werden, angeordnet. Verwirbelungsstrukturen umfassen beispielsweise zylindrische Strukturen, die sich zwischen der Deckplatte der Abdeckung und der Oberfläche der Applikationsseite des Temperierkörpers erstrecken. Verwirbelungsstrukturen können die Wärmeübertragung zwischen Temperierkörper und Übertragungsmedium gegenüber einer unverwirbelten Strömungsführung erhöhen oder die Durchströmung von Kanälen verbessern.

Bei typischen Ausführungsformen umfasst der Temperierkörper Anschlüsse zur Zu-und Abführung des Fluids und Röhren zur Führung des Fluids. Typischerweise sind die Anschlüsse an der Primärseite des Temperierkörpers angeordnet. Typischerweise sind die Röhren mit Kammern in dem Temperierkörper verbunden oder werden durch die Kammern unterbrochen. Die Kammern können insbesondere zur gleichmäßigen Beaufschlagung typischerweise paralleler Röhren mit dem Fluid vorgesehen sein. Typischerweise werden die Röhren von dem Fluid parallel oder in Reihe durchströmt.

Typischerweise umfasst der Temperierkörper oder die Abdeckung Anschlüsse zur Zu- und Abführung des Übertragungsmediums. In einer typischen Ausführungsform sind die Anschlüsse an einander gegenüberliegenden Ecken einer Abdeckung angeordnet. Typischerweise sind die Anschlüsse so gestaltet, dass ein zumindest im Wesentlichen vollständiges Entleeren eines mit Übertragungsmedium gefüllten Hohlraumes vor dem Lösen der Abdeckung möglich ist.

Bei typischen Ausführungsformen ist der Temperierkörper mehrteilig. Der Temperierkörper ist typischerweise durch das Verbinden eines oder mehrerer Metallblöcke, insbesondere durch das Verbinden mehrerer bearbeiteter Metallblöcke, mit Anschlüssen zur Zu- und Abführung des Übertragungsmediums oder des Fluids, Röhren zur Führung des Fluids, strömungsführenden Strukturen oder Verwirbelungsstrukturen hergestellt.

Bei typischen Ausführungsformen ist der Temperierkörper durch Bearbeiten eines Metallblocks oder durch ein additives Fertigungsverfahren wie beispielsweise einem Metall-3D-Druck insbesondere einteilig hergestellt. Es können weitere Bearbeitungsschritte oder weitere Teile oder Blöcke vorhanden sein. Bei typischen Ausführungsformen sind die strömungsführenden Strukturen an der Applikationsseite des Temperierkörpers oder die Röhren zur Führung des Fluids an der Primärseite des Temperierkörpers durch ein Material-abtragendes Verfahren eines Metallblocks des Temperierkörpers hergestellt. Typische Material-abtragende Verfahren, welche verwendet werden, sind beispielsweise Fräsen oder Erodieren.

Bei typischen Ausführungsformen umfasst der Temperierkörper zumindest einen ersten Metallblock, und einen zweiten Metallblock. Der erste Metallblock und der zweite Metallblock sind typischerweise durch ein Fügeverfahren miteinander verbunden, insbesondere miteinander verlötet oder verschraubt oder verschweißt, beispielsweise durch Diffusionsschweißen. Typischerweise sind in dem ersten Metallblock oder in dem zweiten Metallblock an der dem anderen Metallblock zugewandten Seite Nuten mit einem Material-abtragenden Verfahren eingearbeitet.

Die Nuten sind typischerweise so ausgeführt, dass sie die Röhren oder Kammern zur Führung des Fluids bilden. Die Röhren können bei typischen Ausführungsbeispielen auch als Fluidkanäle bezeichnet werden. Bei Ausführungsformen sind die Nuten zur Aufnahme von einsetzbaren Röhren, beispielsweise von Microchannels, ausgeführt. Bei weiteren Ausführungsformen sind die Nuten frei von weiteren Einbauten, beispielsweise frei von Microchannels.

Typischerweise umfasst der Temperierkörper einen Metallblock, der an der Applikationsseite des Temperierkörpers eine Oberfläche zum Kontakt des Temperierkörpers mit dem Übertragungsmedium bereitstellt. Bei einer typischen Ausführungsform sind die strömungsführenden Strukturen an der Oberfläche durch Fräsen oder Erodieren des Metallblocks hergestellt. Bei weiteren typischen Ausführungsformen sind die strömungsführenden Strukturen als eigene Teile hergestellt und nachträglich mit dem Metallblock durch ein Fügeverfahren verbunden, insbesondere mit dem Metallblock verlötet oder verschweißt.

Bei typischen Ausführungsformen umfasst die Vorrichtung einen Temperaturfühler, der mit einer Temperaturanzeige oder einer Regelungselektronik verbunden werden kann. Typischerweise ist ein Temperaturfühler zur Bestimmung der Temperatur des Fluids an dem Temperierkörper angebracht. Typischerweise ist ein Temperaturfühler zur Bestimmung der Temperatur des Übertragungsmediums an dem Temperierkörper oder an der Abdeckung angebracht.

Bei typischen Ausführungsformen weist die Abdeckung eine Dichtung auf, welche den Hohlraum an der Grenze zwischen der an dem Temperierkörper befestigten Abdeckung und dem Temperierkörper abdichtet. Typischerweise weist die Abdeckung zumindest eine der folgenden Dichtungen auf: Eine Labyrinthdichtung, eine Spaltdichtung, eine Dichtung aus einem dichtenden Material wie beispielsweise Kunststoff, Metall oder organische oder anorganische Materialien, eine Flachdichtung, oder eine stoffschlüssige Dichtung. Typischerweise ist die Dichtung an der Abdeckung, insbesondere an dem dem Temperierkörper zugewandten Rand der Randplatte der Abdeckung, ausgeführt. Bei Ausführungsformen ist die Dichtung an der Applikationsseite des Temperierkörpers oder als eigenes Element ausgeführt.

Typischerweise ist die Abdeckung gegen Wärmeverluste aus dem Hohlraum nach außen hin isoliert. Die Abdeckung kann ein isolierendes Mehrkammersystem, insbesondere mit Unterdruck in dem Mehrkammersystem, ein isolierendes Mehrschichtsystem oder eine isolierende Beschichtung aufweisen.

Bei typischen Ausführungsformen ist die Abdeckung vom Temperierkörper lösbar oder am Temperierkörper insbesondere wiederholt befestigbar. Typischerweise ist die Abdeckung beschädigungsfrei vom Temperierkörper lösbar. In einer typischen Ausführungsform ist die Abdeckung wiederholbar lösbar vom Temperierkörper. Eine vom Temperierkörper lösbar ausgeführte Abdeckung kann den Vorteil bieten, dass typische erfindungsgemäße Vorrichtungen keimfrei gehalten werden können, insbesondere durch Trocknen und Reinigen der Applikationsseite des Temperierkörpers bei gelöster Abdeckung.

Bei typischen Ausführungsformen umfasst die Vorrichtung zur Temperierung ein Verbindungselement zur lösbaren Befestigung der Abdeckung an dem Temperierkörper. Das Verbindungselement umfasst insbesondere eine Schraube, einen Kleber, ein Klemmmittel oder einen Magneten. In weiteren Ausführungsformen ist die Abdeckung an dem Temperierkörper, insbesondere zusätzlich zu der Befestigung mit einem Verbindungsmittel, durch Unterdruck in dem von dem Temperierkörper und der Abdeckung gebildeten Hohlraum im Vergleich zu der Umgebung oder durch Adhäsionskräfte zwischen der Abdeckung und dem Temperierkörper befestigt.

Bei typischen Ausführungsformen sind der Temperierkörper und die Abdeckung aus unterschiedlichen Materialien hergestellt. Typischerweise ist der Temperierkörper zumindest im Wesentlichen aus Metall hergestellt. Typischerweise ist der Temperierkörper zu mindestens 90%, oder zu mindestens 80%, oder zu mindestens 70% aus Metall. Die Angaben beziehen sich dabei auf Gewichts-%. Bei weiteren typischen Ausführungsformen ist der Temperierkörper zu weniger als 100% aus Metall, oder zu weniger als 50% aus Metall. Ist der Temperierkörper mehrteilig, können auch Teile des Temperierkörpers aus unterschiedlichen Materialien hergestellt sein. Typischerweise sind zumindest ein Metallblock, der eine Oberfläche zum Kontakt des Temperierkörpers mit dem Übertragungsmedium bereitstellt, oder gegebenenfalls an dem Metallblock angeordnete strömungsführende Strukturen aus besonders wärmeleitfähigen Materialien hergestellt. Typische Metalle, die Verwendung finden können, sind Aluminiumlegierungen, insbesondere solche mit einer Oberflächenveredelung, Messing oder Kupfer. Typische Kunststoffe umfassen beispielsweise Polymere wie PP oder PE in verschiedenen Ausführungen.

Typischerweise ist die Abdeckung zumindest im Wesentlichen aus einem Kunststoff oder zumindest im Wesentlichen aus Glas gefertigt. Typischerweise ist die Abdeckung zu mindestens 90%, oder zu mindestens 80%, oder zu mindestens 70% aus Kunststoff. Die Angaben beziehen sich dabei auf Gewichts-%. Bei weiteren typischen Ausführungsformen ist die Abdeckung zu weniger als 100% aus Kunststoff, oder zu weniger als 50% aus Kunststoff. Typischerweise ist die Abdeckung zu mindestens 90%, oder zu mindestens 80%, oder zu mindestens 70% aus Glas. Bei weiteren typischen Ausführungsformen ist die Abdeckung zu weniger als 100% aus Glas, oder zu weniger als 50% aus Glas.

Bei typischen Ausführungsformen ist die Abdeckung zur einmaligen Nutzung ausgebildet. Bei einer einmaligen Nutzung ist die Abdeckung beispielsweise als Wegwerfartikel oder Einmalartikel ausgebildet. Dadurch kann eine zuverlässige Einhaltung von Hygienestandards erreicht werden. Insbesondere ist die zur einmaligen Nutzung vorgesehene Abdeckung nicht autoklavierbar. Typischerweise ist die Abdeckung steril oder weitgehend keimfrei hergestellt. Bei weiteren Ausführungsformen ist die Abdeckung zur mehrfachen Nutzung ausgebildet, insbesondere autoklavierbar aufgebaut. Auf diese Weise lässt sich die Abdeckung mehrmals verwenden, wodurch Abfall eingespart werden kann.

Bei typischen Ausführungsformen eines Verfahrens zur Temperierung, insbesondere zur extrakorporalen Temperierung von Patienten, wird eine erste Abdeckung an einem Temperierkörper befestigt. Zum Verwenden der Vorrichtung zur Temperierung eines ersten Übertragungsmediums wird typischerweise ein Primärkreis, der ein Fluid bereitstellt, an Anschlüssen zur Zu- und Abführung des Fluids an der Primärseite des Temperierkörpers angeschlossen. Typischerweise wird ein Sekundärkreis, der das erste Übertragungsmedium bereitstellt, an Anschlüssen zur Zu- und Abführung des ersten Übertragungsmediums an der Applikationsseite des Temperierkörpers angeschlossen. Typischerweise werden die Röhren des Temperierkörpers mit dem Fluid befüllt oder von dem Fluid durchströmt. Typischerweise wird der Hohlraum, der von dem Temperierkörper und der Abdeckung gebildet wird, mit dem ersten Übertragungsmedium befüllt und von dem ersten Übertragungsmedium durchströmt.

Bei typischen Verfahren umfasst das Verwenden der Vorrichtung zur Temperierung des ersten Übertragungsmediums ein Verdampfen des Fluids in den Röhren des Temperierkörpers auf eine Kühlanforderung hin. Alternativ oder zusätzlich kann bei typischen Verfahren ein Verflüssigen des Fluids in den Röhren auf eine Wärmeanforderung hin durchgeführt werden.

Typischerweise wird nach dem Verwenden der Vorrichtung zur Temperierung des ersten Übertragungsmediums die erste Abdeckung von dem Temperierkörper gelöst, insbesondere nach dem Ablassen des ersten Übertragungsmediums. Typischerweise wird der Temperierkörper nach dem Lösen der ersten Abdeckung gereinigt, insbesondere die Applikationsseite des Temperierkörpers. Das Reinigen erfolgt typischerweise mit sterilisierenden oder desinfizierenden Reinigungsmitteln. Bei typischen Ausführungsformen umfasst das Reinigen ein Trocknen, insbesondere durch Erwärmen des Temperierkörpers.

Typischerweise wird dann eine zweite Abdeckung an dem Temperierkörper befestigt. Typischerweise wird ein Sekundärkreis, der zweites Übertragungsmedium bereitstellt, an Anschlüssen zur Zu- und Abführung des zweiten Übertragungsmediums an der Applikationsseite des Temperierkörpers angeschlossen. Bei typischen Ausführungsformen des Verfahrens wird die Vorrichtung anschließend zur Temperierung des zweiten Übertragungsmediums verwendet.

Bei typischen Ausführungsformen werden die erste Abdeckung und die zweite Abdeckung jeweils nach dem Verwenden entsorgt. Bei weiteren typischen Ausführungsformen werden die erste Abdeckung und die zweite Abdeckung nach dem Verwenden gereinigt oder sterilisiert, beispielsweise autoklaviert, und werden wiederverwendet.

Typische Vorteile von Ausführungsformen umfassen beispielsweise, dass die strömungsführenden Strukturen an dem Temperierkörper oder an der Abdeckung anwendungsspezifisch ausgeführt werden können. Es können unterschiedliche Temperieraufgaben bewältigt werden, nicht nur eine extrakorporale Temperierung von Patienten oder von Körperflüssigkeiten. Es können auch andere Medien temperiert werden. Ein weiterer Vorteil typischer Ausführungsformen kann sein, dass die strömungsführenden Strukturen eine hohe Wärmeübertragung zwischen der Primärseite des Temperierkörpers, insbesondere dem Fluid, und dem Übertragungsmedium gewährleisten. Ein Vorteil typischer Ausführungsformen kann die lösbare Befestigung der Abdeckung und die gute Zugänglichkeit der Oberfläche der Applikationsseite des Temperierkörpers sein. Typische Ausführungsformen bieten die Möglichkeit zu einer effizienten Reinigung oder zur Ausführung der Abdeckung oder anderer Teile der beanspruchten Vorrichtung als Wegwerfprodukt.

### Kurze Beschreibung der Zeichnungen

Nachfolgend werden Ausführungsbeispiele der Erfindung anhand von Zeichnungen näher erläutert. Darin zeigen:
- Fig. 1: einen schematischen Aufbau eines Systems mit einer typischen Vorrichtung zur Temperierung, insbesondere zur extrakorporalen Temperierung von Patienten oder zur extrakorporalen Temperierung von Körperflüssigkeiten;
- Fig. 2: eine typische Vorrichtung in einer schematischen Explosivdarstellung; und
- Fig. 3: ein Verfahren in einer typischen Ausführungsform.

### Beschreibung der in den Figuren gezeigten Ausführungsbeispiele

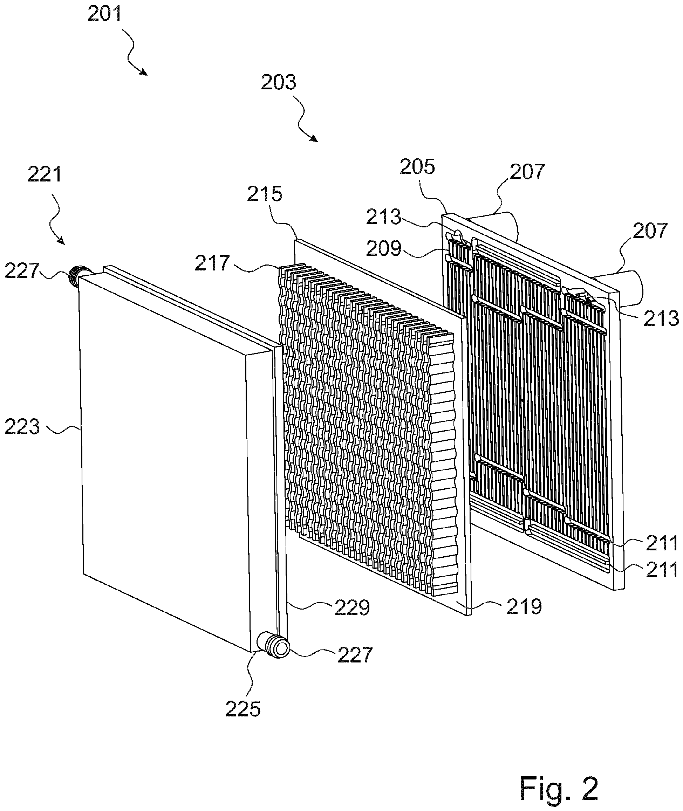

Nachfolgend werden typische Ausführungsbeispiele der Erfindung beschrieben, wobei für gleiche oder ähnliche Teile teilweise gleiche Bezugszeichen verwendet werden und unter Umständen nicht mit jeder Figur nochmals erläutert werden. Die Erfindung ist nicht auf die nachfolgend beschriebenen typischen Ausführungsformen beschränkt. Zur Übersichtlichkeit sind teilweise nicht alle jeweiligen Merkmale mit einem Bezugszeichen versehen, beispielsweise die Nuten mit dem Bezugszeichen 209 der Fig. 2.

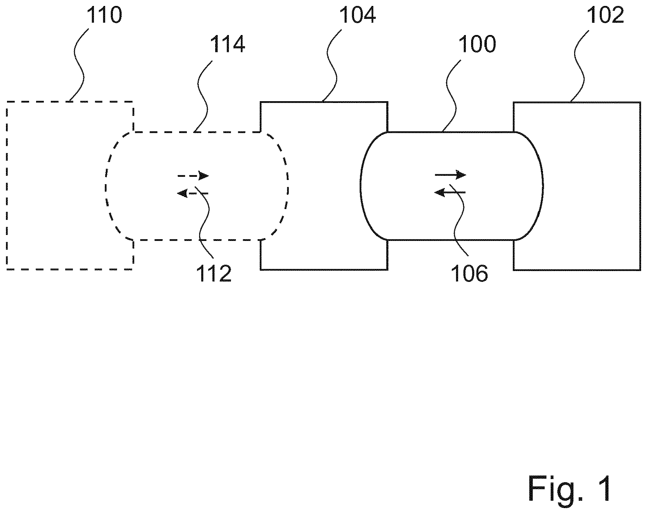

In der Fig. 1 ist schematisch eine Vorrichtung 100 gezeigt, welche eine typische Ausführungsform der Erfindung ist. Typischerweise umfasst die Vorrichtung 100 einen Temperierkörper und eine an dem Temperierkörper befestigte Abdeckung, wobei der Temperierkörper eine Primärseite, welche eine Führung für ein Fluid eines Primärkreises 102 bereitstellt, und eine Applikationsseite aufweist, welche eine Oberfläche zum Kontakt des Temperierkörpers mit einem Übertragungsmedium eines Sekundärkreises 104 bereitstellt. Der Temperierkörper der Vorrichtung 100 ermöglicht die Wärmeübertragung 106 zwischen einem Fluid des Primärkreises 102 und einem Übertragungsmedium des Sekundärkreises 104.

Die Formulierung "Wärmeübertragung" umfasst wie allgemein in dieser Beschreibung einen Transport von Wärme in zumindest eine beliebige oder in beide Richtungen, insbesondere eine Heizung oder eine Kühlung des Übertragungsmediums.

Die Wärmeübertragung wird typischerweise durch den Kontakt des Fluids mit dem Temperierkörper an der Primärseite des Temperierkörpers, den Wärmetransport in dem Temperierkörper und durch den Kontakt des Übertragungsmediums mit dem Temperierkörper an der Applikationsseite des Temperierkörpers realisiert. Bei typischen Ausführungsformen wird die Leistung oder Präzision der Wärmeübertragung durch strömungsführende Strukturen, insbesondere durch wärmeleitende strömungsführende Strukturen, gegenüber ungeführter Strömung erhöht.

Das Übertragungsmedium, im dargestellten Beispiel der Fig. 1 eine isotonische 0,9%-NaCl-Lösung, des Sekundärkreises 104 überträgt Wärme zu einem Wärmetauscher 114, der beispielsweise Teil eines Oxygenators ist. In dem Wärmetauscher 114 wird durch einen Wärmeaustausch 112 Wärme zwischen dem Übertragungsmedium des Sekundärkreises 104 und einer Körperflüssigkeit bzw. Flüssigkeit 110, beispielsweise Blut eines Patienten, übertragen.

Mit typischen Vorrichtungen wird ein Patient oder ein Tier, dessen Blutkreislauf zumindest teilweise durch den Oxygenator geführt wird, temperiert, d.h. gekühlt oder erwärmt.

Fig. 2 zeigt eine typische Vorrichtung 201 zur Temperierung mit einem Temperierkörper 203 und einer an dem Temperierkörper befestigten Abdeckung 221 in Explosivdarstellung. In der beispielhaften Ausführungsform der Fig. 2 umfasst der Temperierkörper 203 einen ersten Metallblock 205 und einen zweiten Metallblock 215, die miteinander verschweißt sind.

An einer Primärseite des Temperierkörpers 203 sind Primäranschlüsse 207 als Anschlüsse zur Zu- und Abführung eines Fluids angebracht. In der gezeigten Ausführungsform sind die Primäranschlüsse 207 an dem ersten Metallblock 205 angebracht.

Der Temperierkörper 203 enthält Röhren 209 zur Führung des Fluids. Die Primäranschlüsse 207 sind über Durchführungen 213 mit den Röhren 209 verbunden. Die Durchführungen 213 sind, wie in Fig. 2 beispielhaft gezeigt, durch den ersten Metallblock 205 gefräst.

Die Primäranschlüsse 207 des Temperierkörpers 203 sind durch die Röhren 209 miteinander verbunden. Bei der Ausführungsform der Fig. 2 sind die Röhren 209 mit Kammern 211 verbunden oder werden durch die Kammern 211 unterbrochen. Die Kammern 211 sind zur gleichmäßigen Beaufschlagung der parallelen Röhren 209 mit dem Fluid vorgesehen.

Bei dem Ausführungsbeispiel der Fig. 2 sind Nuten und Vertiefungen in den ersten Metallblock 205 eingefräst oder erodiert. Die Nuten und Vertiefungen sind durch das Verbinden des ersten Metallblocks 205 mit dem zweiten Metallblock 215 abgedeckt und so zu Röhren 209 beziehungsweise Kammern 211 verschlossen.

Der Temperierkörper 203 weist strömungsführende Strukturen 217 an einer Oberfläche 219 einer Applikationsseite des Temperierkörpers 203 auf. Bei dem Ausführungsbeispiel der Fig. 2 sind die strömungsführenden Strukturen 217 als gewellte Rippen ausgeführt. Bei dem Ausführungsbeispiel der Fig. 2 sind die strömungsführenden Strukturen 217 aus dem zweiten Metallblock 215 herausgefräst.

Eine Eignung zu umfassender Reinigung ergibt sich bei Ausführungsformen beispielsweise durch die Möglichkeit, die Abdeckung zu lösen und den Temperierkörper zu sterilisieren und zu trocknen. Typische Ausführungsformen umfassen Temperierkörper oder Abdeckung, welche zumindest im Wesentlichen frei von hinterschnittenen Strukturen sind. Insbesondere sind die Rippen Hinterschneidungs-los an der Abdeckung oder am Temperierkörper ausgeführt.

Die Abdeckung 221 der Vorrichtung 201 ist lösbar an dem Temperierkörper 203 befestigt. Bei dem Ausführungsbeispiel der Fig. 2 umfasst die Abdeckung 221 eine Deckplatte 223, die im Wesentlichen parallel zu der Oberfläche 219 der Applikationsseite des Temperierkörpers 203 verläuft, eine Randplatte 225, die sich zwischen dem Rand der Deckplatte 223 und der Oberfläche 219 der Applikationsseite des Temperierkörpers 203 erstreckt, Sekundäranschlüsse 227 als Anschlüsse zur Zu- und Abführung eines Übertragungsmediums und ein Verbindungselement 229 zum Befestigen der Abdeckung 221 an dem Temperierkörper 203.

Die strömungsführenden Strukturen 217 des Ausführungsbeispiels der Fig. 2 sind so ausgeführt, dass sie sich von der Oberfläche 219 der Applikationsseite des Temperierkörpers 203 bis zu der Deckplatte 223 der Abdeckung 221 erstrecken. Die strömungsführenden Strukturen 217 bilden zur Führung des Übertragungsmediums parallel verlaufende Kanäle.

Bei einer Verwendung der Vorrichtung 201 in der typischen Ausführungsform der Fig. 1 strömt Übertragungsmedium durch einen ersten Sekundäranschluss der Sekundäranschlüsse 227 in den Hohlraum, der von dem Temperierkörper 203 und der Abdeckung 221 gebildet wird, durchströmt parallel die von den strömungsführenden Strukturen 217 gebildeten Kanäle und tritt an einem zweiten Sekundäranschluss der Sekundäranschlüsse 227 aus dem Hohlraum aus.

In der beispielhaften Ausführungsform der Fig. 2 ist die Abdeckung 221 mittels des Verbindungselements 229, das als leistenförmiges Klemmmittel an der Randplatte 225 ausgeführt ist, lösbar auf den Temperierkörper 203 gesteckt. Typischerweise weist die Abdeckung 221 eine Dichtung auf, die beispielsweise an dem der Oberfläche 219 des Temperierkörpers 203 zugewandten Rand der Randplatte 225 angeordnet ist.

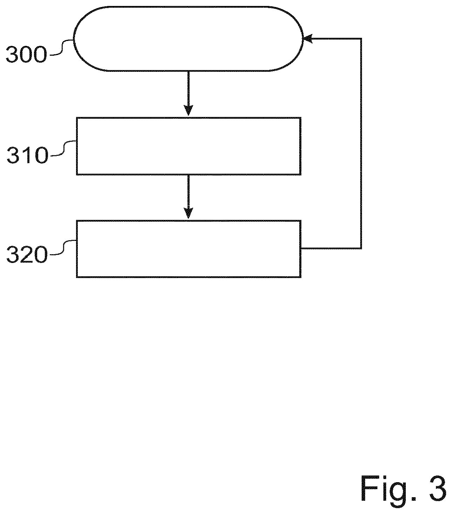

In der Fig. 3 ist schematisch ein typisches Verfahren zur extrakorporalen Temperierung von Patienten, mit einer Übertragung von Wärme zwischen einem Fluid und einem Übertragungsmedium gezeigt. Das Verfahren kann beispielsweise mit einer typischen Ausführungsform einer Vorrichtung, wie sie im Zusammenhang mit den Figuren 1 und 2 erläutert wird, durchgeführt werden.

Bei dem beispielhaften Verfahren der Fig. 3 wird bei 300 eine erste Abdeckung mittels eines Klemmmittels als Verbindungselement lösbar an einem Temperierkörper befestigt.

Die lösbare Befestigung ist typischerweise beschädigungsfrei lösbar. Typischerweise ist ein von dem Temperierkörper und der ersten Abdeckung gebildeter erster Hohlraum durch die lösbare Befestigung gegen ein unkontrolliertes Austreten von Übertragungsmedium abgedichtet.

Bei 310 wird eine Vorrichtung, die den Temperierkörper und die erste Abdeckung umfasst, verwendet, um ein erstes Übertragungsmedium in der Vorrichtung mittels des durch den Temperierkörper geführten Fluids zu temperieren. Die Temperierung erfolgt durch ein Verdampfen des Fluids auf eine Kühlanforderung hin und ein Verflüssigen des Fluids auf eine Wärmeanforderung hin. Je nach Bedarf wird die Wärmeleistung der Primärseite so geregelt, dass sich die gewünschte Temperatur in dem Sekundärkreis einstellt. Die Temperatur in dem Primärkreis oder die Temperatur in dem Sekundärkreis kann dabei jeweils je nach Anforderung um einige Grad niedriger oder höher sein.

Bei 320 wird die erste Abdeckung von dem Temperierkörper beschädigungsfrei gelöst, insbesondere nach dem Ablassen des ersten Übertragungsmediums aus dem ersten Hohlraum.

Typischerweise wird nach 320 der Temperierkörper, insbesondere eine Oberfläche des Temperierkörpers, die in Kontakt mit dem ersten Übertragungsmedium stand, gereinigt. Typischerweise umfasst das Reinigen eine Sterilisierung und ein Trocknen des Temperierkörpers oder der Oberfläche des Temperierkörpers, die in Kontakt mit dem ersten Übertragungsmedium stand. Bei einer typischen Ausführungsform des Verfahrens wird die erste Abdeckung entsorgt. Bei weiteren Ausführungsformen wird die erste Abdeckung zur Wiederverwendung gereinigt und sterilisiert.

Das Verfahren springt anschließend für den nächsten Patienten zu 300 zurück, wobei dann im zweiten Durchlauf eine zweite Abdeckung und ein zweites Übertragungsmedium zum Einsatz kommen, so dass keine im ersten Durchlauf in das erste Übertragungsmedium eingetragenen Keime zu einer Verunreinigung führen. Ein dritter und weitere Durchläufe des Verfahrens können zur Temperierung von weiteren Patienten erfolgen.

Die Wiederholung kann auch mehrmals oder bei weiteren Ausführungsbeispielen mit einer gereinigten und sterilisierten Abdeckung erfolgen.

## Patentansprüche

1. Vorrichtung (100, 201) zur Temperierung, insbesondere zur extrakorporalen Temperierung von Patienten und/oder zur extrakorporalen Temperierung von Körperflüssigkeiten , mit einer Übertragung von Wärme zwischen einem Fluid und einem Übertragungsmedium, mit
- einem Temperierkörper (203) mit einer Primärseite, welche eine Führung für das Fluid bereitstellt, und mit einer Applikationsseite, welche eine Oberfläche (219) zum Kontakt des Temperierkörpers (203) mit dem Übertragungsmedium bereitstellt;
- einer an dem Temperierkörper (203) befestigten Abdeckung (221), welche an der Applikationsseite mit dem Temperierkörper (203) einen Hohlraum bildet, der von dem Übertragungsmedium durchströmt werden kann;
- wobei die Abdeckung (221) lösbar an dem Temperierkörper (203) befestigt ist; und
- wobei an dem Temperierkörper (203) und/oder an der Abdeckung (221) strömungsführende Strukturen (217) zur Führung des Übertragungsmediums angeordnet sind.

2. Vorrichtung nach Anspruch 1, wobei die strömungsführenden Strukturen bei Befestigung der Abdeckung (221) an dem Temperierkörper (203) von der Oberfläche (219) der Applikationsseite bis zu einer Deckplatte (223) der Abdeckung (221) reichen.

3. Vorrichtung nach einem der vorhergehenden Ansprüche, wobei der Temperierkörper (203) und/oder die Abdeckung (221) Verwirbelungsstrukturen zur Verwirbelung des Übertragungsmediums aufweisen.

4. Vorrichtung nach einem der vorhergehenden Ansprüche, wobei der Temperierkörper (203) Anschlüsse (207) zur Zu- und Abführung des Fluids und Röhren (209) zur Führung des Fluids umfasst.

5. Vorrichtung nach einem der vorhergehenden Ansprüche, wobei der Temperierkörper (203) und/oder die Abdeckung (221) Anschlüsse (227) zur Zu- und Abführung des Übertragungsmediums umfassen.

6. Vorrichtung nach einem der vorhergehenden Ansprüche, wobei der Temperierkörper (203) mehrteilig ist.

7. Vorrichtung nach einem der vorhergehenden Ansprüche, wobei der Temperierkörper (203) durch Bearbeiten eines Metallblocks (205, 215) und/oder durch ein additives Fertigungsverfahren insbesondere einteilig hergestellt ist.

8. Vorrichtung nach einem der vorhergehenden Ansprüche, wobei die strömungsführenden Strukturen (217) an der Applikationsseite des Temperierkörpers (203) und/oder die Röhren (209) zur Führung des Fluids an der Primärseite des Temperierkörpers (203) durch ein Material-abtragendes Verfahren eines Metallblocks (205, 215) des Temperierkörpers (203) hergestellt sind.

9. Vorrichtung nach einem der vorhergehenden Ansprüche, wobei die Abdeckung (221) eine Dichtung aufweist, welche den Hohlraum an der Grenze zwischen der an dem Temperierkörper (203) befestigten Abdeckung (221) und dem Temperierkörper (203) abdichtet.

10. Vorrichtung nach einem der vorhergehenden Ansprüche, mit einem Verbindungselement (229) zur lösbaren Befestigung der Abdeckung (221) an dem Temperierkörper (203).

11. Vorrichtung nach einem der vorhergehenden Ansprüche, wobei der Temperierkörper (203) und die Abdeckung (221) aus unterschiedlichen Materialien hergestellt sind.

12. Vorrichtung nach einem der vorhergehenden Ansprüche, wobei die Abdeckung (221) zur einmaligen Nutzung ausgebildet ist.

13. Vorrichtung nach einem der vorhergehenden Ansprüche, wobei die Abdeckung (221) zumindest im Wesentlichen aus einem Kunststoff oder zumindest im Wesentlichen aus Glas gefertigt ist.

14. Verfahren zur Temperierung, insbesondere zur extrakorporalen Temperierung von Patienten und/oder zur extrakorporalen Temperierung von Körperflüssigkeiten unter Verwendung eines Temperierkörpers und zweier Abdeckungen entsprechend einer Vorrichtung nach einem der Ansprüche 1 bis 13, mit
- Befestigen (300) einer ersten Abdeckung an dem Temperierkörper,
- Verwenden (310) der Vorrichtung zur Temperierung des Übertragungsmediums,
- Lösen (320) der ersten Abdeckung von dem Temperierkörper,
- Befestigen einer zweiten Abdeckung an dem Temperierkörper,
- Verwenden der Vorrichtung zur Temperierung des Übertragungsmediums.

15. Verfahren nach Anspruch 14, wobei die erste Abdeckung und die zweite Abdeckung jeweils nach dem Verwenden entsorgt werden.
